# EUROPEAN PATENT SPECIFICATION

(11) **EP 1 338 252 B1**
(45) Date of publication and mention of the grant of the patent: **07.07.2010**
(21) Application number: 03250106.6
(22) Date of filing: 08.01.2003
(51) Int. Cl.: A61F 2/06

(54) **Sealing prosthesis**
Abdichtende Prothese
Prothèse etanche

(30) Priority: 08.01.2002 US 41366
(43) Date of publication of application: 27.08.2003
(73) Proprietor: Cordis Corporation, Miami Lakes, Florida 33014 (US)
(72) Inventor: Depalma, Donald F., Weston, FL 33326 (US); Letendre, Robert P., Hialeah, FL 33015 (US)
(74) Representative: Belcher, Simon James

(56) References cited:
- EP-A- 0 947 179
- US-A1- 2002 058 985
- US-B1- 6 325 819

## Description

The present invention relates to devices for repairing aneurysms, and more particularly, to intraluminally and/or percutaneously delivered devices for repairing aneurysms, such as abdominal aortic aneurysms and thoracic aortic aneurysms.

An aneurysm is an abnormal dilation of a layer or layers of an arterial wall, usually caused by a systemic collagen synthetic or structural defect. An abdominal aortic aneurysm is an aneurysm in the abdominal portion of the aorta, usually located in or near one or both of the two iliac arteries or near the renal arteries. The aneurysm often arises in the infrarenal portion of the diseased aorta, for example, below the kidneys. A thoracic aortic aneurysm is an aneurysm in the thoracic portion of the aorta. When left untreated, the aneurysm may rupture, usually causing rapid fatal haemorrhaging.

Aneurysms may be classified or typed by their position as well as by the number of aneurysms in a cluster. Typically, abdominal aortic aneurysms may be classified into five types. A Type I aneurysm is a single dilation located between the renal arteries and the iliac arteries. Typically, in a Type I aneurysm, the aorta is healthy between the renal arteries and the aneurysm and between the aneurysm and the iliac arteries.

A Type II A aneurysm is a single dilation located between the renal arteries and the iliac arteries. In a Type II A aneurysm, the aorta is healthy between the renal ar 1 eries and the aneurysm, but not healthy between the aneurysm and the iliac arteries. In other words, the dilation extends to the aortic bifurcation. A Type II B aneurysm comprises three dilations. One dilation is located between the renal arteries and the iliac arteries. Like a Type II A aneurysm, the aorta is healthy between the aneurysm and the renal arteries, but not healthy between the aneurysm and the iliac arteries. The other two dilations are located in the iliac arteries between the aortic bifurcation and the bifurcations between the external iliacs and the internal iliacs. The iliac arteries are healthy between the iliac bifurcation and the aneurysms. A Type II C aneurysm also comprises three dilations. However, in a Type II C aneurysm, the dilations in the iliac arteries extend to the iliac bifurcation.

A Type III aneurysm is a single dilation located between the renal arteries and the iliac arteries. In a Type III aneurysm, the aorta is not healthy between the renal arteries and the aneurysm. In other words, the dilation extends to the renal arteries.

A ruptured abdominal aortic aneurysm is presently the thirteenth leading cause of death in the United States. The routine management of abdominal aortic aneurysms has been surgical bypass, with the placement of a graft in the involved or dilated segment. Although resection with a synthetic graft via transperitoneal or retroperitoneal approach has been the standard treatment, it is associated with significant risk. For example, complications include perioperative myocardial ischemia, renal failure, erectile impotence, intestinal ischemia, infection, lower limb ischemia, spinal cord injury with paralysis, aortaenteric fistula, and death. Surgical treatment of abdominal aortic aneurysms is associated with an overall mortality rate of five percent in asymptomatic patients, sixteen to nineteen percent in symptomatic patients, and is as high as fifty percent in patients with ruptured abdominal aortic aneurysms.

Disadvantages associated with conventional surgery, in addition to the high mortality rate, include an extended recovery period associated with the large surgical incision and the opening of the abdominal cavity, difficulties in suturing the graft to the aorta, the loss of the existing thrombosis to support and reinforce the graft, the unsuitability of the surgery for many patients having abdominal aortic aneurysms, and the problems associated with performing the surgery on an emergency basis after the aneurysm has ruptured. Further, the typical recovery period is from one to two weeks in the hospital, and a convalescence period at home from two to three months or more, if complications ensue. Since many patients having abdominal aortic aneurysms have other chronic illnesses, such as heart, lung, liver and/or kidney disease, coupled with the fact that many of these patients are older, they are less than ideal candidates for surgery.

The occurrence of aneurysms is not confined to the abdominal region. While abdominal aortic aneurysms are generally the most common, aneurysms in other regions of the aorta or one of its branches are possible. For example, aneurysms may occur in the thoracic aorta. As is the case with abdominal aortic aneurysms, the widely accepted approach to treating an aneurysm in the thoracic aorta is surgical repair, involving replacing the aneurysmal segment with a prosthetic device. This surgery, as described above, is a major undertaking, with associated high risks and with significant mortality and morbidity.

Over the past five years, there has been a great deal of research directed at developing less invasive, percutaneous, e.g., catheter directed, techniques for the treatment of aneurysms, specifically abdominal aortic aneurysms. This has been facilitated by the development of vascular stents, which can and have been used in conjunction with standard or thin-wall graft material in order to create a stent-graft or endograft. The potential advantages of less invasive treatments have included reduced surgical morbidity and mortality along with shorter hospital and intensive care unit stays.

Stent-grafts or endoprostheses are now FDA approved and commercially available. The delivery procedure typically involves advanced angiographic techniques performed through vascular accesses gained via surgical cutdown of a remote artery, such as the common femoral or brachial arteries. Over a guidewire, the appropriate size introducer will be placed. The catheter and guidewire is passed through the aneurysm, and, with the appropriate size introducer housing a stent-graft, the stent-graft will be advanced along the guidewire to the appropriate position. Typical deployment of the stent-graft device requires withdrawal of an outer sheath while maintaining the position of the stent-graft with an inner-stabilizing device. Most stent-grafts are self-expanding; however, an additional angioplasty procedure, e.g., balloon angioplasty, may be required to secure the position of the stent- graft. Following the placement of the stent-graft, standard angiographic views may be obtained.

Due to the large diameter of the above-described devices, typically greater than twenty French (3F = 1 mm), arteriotomy closure requires surgical repair. Some procedures may require additional surgical techniques, such as hypogastric artery embolization, vessel ligation, or surgical bypass, in order to adequately treat the aneurysm or to maintain flow to both lower extremities. Likewise, some procedures will require additional, advanced catheter directed techniques, such as angioplasty, stent placement, and embolization, in order to successfully exclude the aneurysm and efficiently manage leaks.

While the above-described endoprostheses represent a significant improvement over conventional surgical techniques, there is a need to improve the endoprostheses, their method of use and their applicability to varied biological conditions. Accordingly, in order to provide a safe and effective alternate means for treating aneurysms, including abdominal aortic aneurysms and thoracic aortic aneurysms, a number of difficulties associated with currently known endoprostheses and their delivery systems must be overcome. One concern with the use of endoprostheses is the prevention of endo-leaks and the disruption of the normal fluid dynamics of the vasculature. Devices using any technology should preferably be simple to position and reposition as necessary, should preferably provide an acute fluid tight seal, and should preferably be anchored to prevent migration without interfering with normal blood flow in both the aneurysmal vessel as well as branching vessels. In addition, devices using the technology should preferably be able to be anchored, sealed, and maintained in bifurcated vessels, tortuous vessels, highly angulated vessels, partially diseased vessels, calcified vessels, odd shaped vessels, short vessels, and long vessels. In order to accomplish this, the endoprostheses should preferably be extendable and re-configurable while maintaining acute and long term fluid tight seals and anchoring positions.

The endoprostheses should also preferably be able to be delivered percutaneously utilizing catheters, guidewires and other devices which substantially eliminate the need for open surgical intervention. Accordingly, the diameter of the endoprostheses in the catheter is an important factor. This is especially true for aneurysms in the larger vessels, such as the thoracic aorta.

EP-A-0947179 discusses a pre-cursor stent for positioning within the infrarenal neck between an abdominal aortic aneurysm and the renal arteries. The stent is designed to be coupled to a graft for directing blood flow.

The sealing prosthesis of the present invention provides a means for overcoming the problems associated with anchoring and/or sealing at least one by-pass prosthesis in an artery as briefly described above.

According to the present invention, there is provided a first prosthesis including a stent, a gasket material supported by or covering the interior and/or exterior surface of the stent or a portion of the stent; the stent and gasket material being configured to seal an aneurysm wherein a portion of the gasket material is positioned at the proximal end of the aneurysm across the diameter of the cross section of said first prosthesis; said portion of gasket material includes a partition and a thread defining a section within the portion; the section having less material than the partition. The section may receive a first second prosthesis; the second prosthesis being configured for establishing a fluid flow channel through the aneurysm.

In a preferred embodiment of the invention, the portion further includes a second thread defining a second section having less material than the partition. The second section may be configured to receive a second second prosthesis.

Some embodiments of the present invention may further comprise a third thread defining a third section having less material than the partition, configured to receive a first third prosthesis, and a fourth thread defining a fourth section, having less material than the partition, configured to receive a second third prosthesis. In these embodiments of the invention, the third prostheses do not pass through the aneurysm and are configured for establishing a fluid flow channel from a proximal portion of the first prosthesis and into a cross artery, such as renal artery.

Any of the prostheses described above may form a component or portion of a system or kit for repairing or bypassing an aneurysm.

The present invention also provides a system for repairing and/or replacing an aneurysm, said system being variously configured and/or assembled using components described in more detail below. Typical systems according to this aspect of the invention includes one first prosthesis or a sealing component, and one or more second prostheses or a fluid flow component. Preferred embodiments of a system of the present invention include a sealing component matingly engaged to two fluid flow path components.

Any of the prostheses, stents, systems, or kits described above may be used in a method for treating an aneurysm. In preferred embodiments of the invention, the prostheses, stents, systems, or kits are used to treat an aortic aneurysm, even more preferably, an abdominal aortic aneurysm.

The method involves delivering and deploying a first prosthesis upstream of an aneurysm, the first prosthesis being adapted to receive a first second prosthesis and at least a second second prosthesis; positioning a proximal end of the first second prosthesis in a proximal end on the first prosthesis, and positioning a distal end of the first second prosthesis in an artery downstream of the aneurysm; and positioning a proximal end of at least a second second prosthesis in a proximal end of the first prosthesis. In some examples of the invention, the method may further include positioning a distal end of the second second prosthesis in an artery downstream of the aneurysm. In further examples of the invention, the method includes anchoring the system using the first second prosthesis in its expanded configuration. The method may further include anchoring the most upstream portion of the system using the first prosthesis.

Other embodiments of the invention will be evident from the description provided below.

### DEFINITIONS

As used herein, aortic aneurysm refers to any failure of a conduit, such as an aortic wall, typically characterized by an undesirable dilation of a portion of the artery, vessel malformation, or an occlusion. The system and structures of the present invention may be used to treat, repair, replace, or bypass any blood vessel (e.g., artery, vein, capillary); any fluid carrying vessel (e,g., lymphatic vessels); any organ or portion thereof that includes a blood or fluid vessel; or any junction between blood vessels, between fluid vessels, and between organs and blood vessels. An exemplary use of a system of the present invention is to repair an aortic aneurysm, and the use of such term is not intended to limit the use of the structures or systems of the, present invention to repair or replace other conduit failures. The prosthesis of the present invention may also be utilized in the thoracic aorta, and may be used to repair thoracic aneurysms or thoracic dissecting aneurysms. Accordingly, use of the term "aortic aneurysm" is intended to relate to and include other aneurysms, including but not limited to both abdominal aortic aneurysms and thoracic aneurysms.

In preferred embodiments of the invention, the system and structures are used to treat, repair, replace, or bypass an abdominal aortic aneurysm. As used herein fluid pathway refers to any *in vivo* structure through which a biological fluid passes. A preferred fluid pathway is an artery. Fluid pathways include, but are not limited to channels formed by an artery, a vein, a capillary, lymph nodes and channels, and arteries, veins, and capillaries within an organ or organelle.

As used herein fluid or biological fluid refers to any fluid produced by an animal, including a human. Exemplary biological fluids include, but are not limited to blood, oxygenated blood, de-oxygenated blood, gastric fluids, amniotic fluid, spinal fluid, and lymph. The preferred fluid is blood or oxygenated blood.

As used herein, conduit typically refers to any structure used to convey a biological fluid. The conduit may be formed of natural or synthetic materials or combinations thereof. Exemplary conduits include but are not limited to an artery, a vein, a capillary, lymph nodes and channels, and arteries, veins, capillaries within an organ or organelle, and a prosthesis or system according to the invention.

As used herein, "biofusion" refers to the ability of cells, proteins, fibrin, and other biological molecules to incorporate into the pore structure of a material, such as a foam or gasket material, or a graft material. It is believed that this feature promotes a long-term stable biological interface that cannot be separated about six weeks after implantation.

The biofusion effect has many advantages. It has the potential to obviate late endo-leakage by preventing areas of non-organized clot from being displaced or recanalised. It is also believed that biofusion creates a connective tissue collar around the prosthesis that may prevent the aortic neck from dilating over time. Restricting neck dilation avoids leakage pathways and implant migration that can be caused by an insufficient fit with the aorta.

As used herein, adapted for communication, communicating, or similar terms refer to any means, structures, or methods for establishing operational association between two elements of the system. Similarly, engaging, adapted to engage, or similar terms refer to means, structures, or methods for contacting a first component, structure, or portion thereof with a second component, structure, or portion thereof. Exemplary structures are shown in the Figures. Typically, all of these terms and phrases refer to at least one structure in or on a first component configured to engage a complementary structure in or on a second component, and the use of these inter-engaging features to link a first prosthesis or component with a second prosthesis or component. The engagement or communication may be matingly (e.g., permanent) and/or releasably (e.g., temporary). In preferred embodiments of the invention, communication or engagement may be fluid tight, substi3ntially fluid tight, or fluid tight to an extent so as to not substantially compromise the intended function of the structure.

For example, a connector may be adapted to receive or connect to a complementary connector on another prosthesis. As used herein, connector refers to any structure used to form a joint or to join itself to another component or portion thereof. These connectors or connections establish a fluid flow path through various elements of the apparatus, assembly, or system. In a preferred embodiment of the invention, the system is intended to establish at least one fluid flow path through a vessel, conduit, organ, or portions thereof. Typical connections include but are not limited to mating connections, such as Luer-type, screw-type, friction-type, or connectors that are bonded together.

As used herein, distal is used in accordance with its ordinary dictionary definition, e.g., referring to a position farthest from the beginning; in human anatomy, this term is commonly equivalent to caudal or inferior. Proximal is used in accordance with its ordinary dictionary definition, e.g., referring to a position nearest the beginning; in human anatomy, this term is commonly equivalent to cranial or superior. The terms distal and proximal are intended to convey opposite ends or portions of a device, channel, element, or structure.

In relation to a fluid flow path, distal will typically refer to a downstream location in the fluid flow path, and proximal will typically refer to an upstream location, unless otherwise specifically noted. Anatomically, distal generally refers to "away from the heart" and proximal generally refers to "toward the heart".

A system for treating an aortic aneurysm according to the present invention typically includes a first prosthesis or precursor stent and at least one second prosthesis. In preferred embodiments of the invention, the components of the system are delivered intraluminally to the site of the aneurysm using a catheter or the like. One skilled in the art will therefore recognize that it is beneficial to deliver the components of the system in an unexpanded or first position, and to deploy the component in its functional location by expanding the component into an expanded or second position. In some embodiments of the invention, the component is self-expanding; that is, once the component is released from its delivery device, the component automatically moves into its open position. In other embodiments of the invention, the component may be expandable using a balloon or the like, as is well known to those skilled in the art. A typical second prosthesis form a fluid flow channel that bypasses the aneurysm. The system may also include at least one-third prosthesis, typically forming a fluid flow path into a cross artery upstream of the aneurysm.

Embodiments of the invention will now be described by way of example with reference to the accompanying drawings, in which:
Figure 1 is an elevation view of a fully deployed aortic repair system made in accordance with the present invention.
Figure 2 is a perspective view of a stent for a first prosthesis, shown for clarity in an expanded state.
Figure 3 is a perspective view of a first prosthesis having a stent covered by a gasket material.
Figure 4 is a side elevation of a second prosthesis having a stent covered by a graft material.
Figure 5 is an elevation view of a fully deployed first prosthesis made in accordance with the present invention and an exemplary delivery system.
Figure 6 is a side cross section of a first prosthesis according to the present invention.
Figure 7a to 7e are a top view of alternate embodiments of a gasket material on a first prosthesis according to the present invention.

### SYSTEM

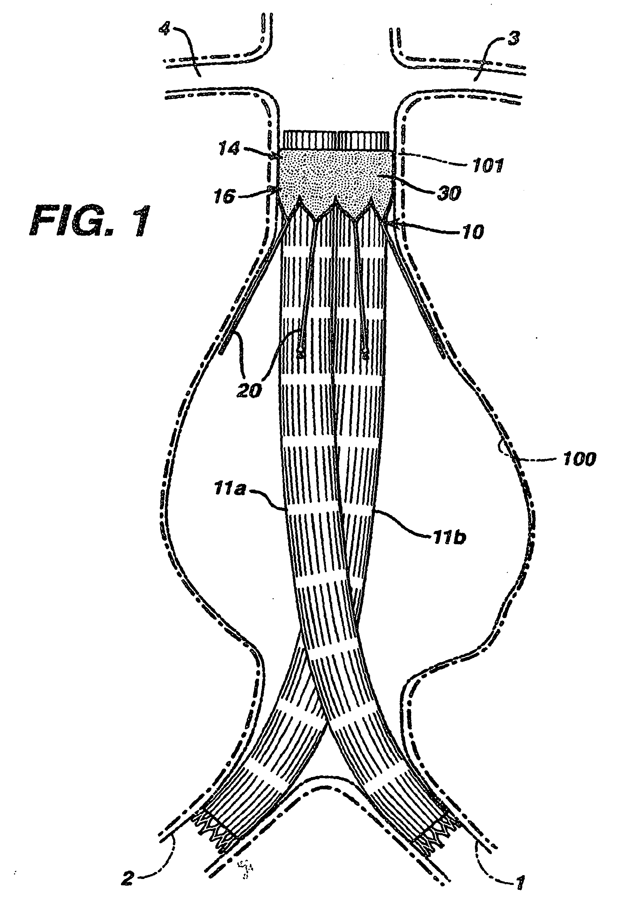

A system according to the present invention may include one or more prostheses. In the exemplary system shown in Figure 1, the system includes a first prosthesis 10 and two-second prostheses 11a and 11b, which, in combination, bypass an aneurysm 100. In preferred embodiments of the invention, a proximal portion of the system may be positioned in a section 101 of an artery upstream of the aneurysm 100, and a distal portion of the system may be positioned in a downstream section of the artery or a different artery.

A prosthesis used in a system in accordance with the present invention typically includes a support, stent or lattice of interconnected struts defining an interior space or lumen having an open proximal end and an open distal end. The lattice also defines an interior surface and an exterior surface. The interior and/or exterior surfaces of the lattice, or a portion of the lattice, may be covered by or support at least one gasket material or graft material.

In preferred embodiments of the invention, a prosthesis is moveable between an expanded or inflated position and an unexpanded or deflated position, and any position therebetween. In some embodiments of the invention, it may be desirable to provide a prosthesis that moves only from fully collapsed to fully expanded. In other embodiments of the invention, it may be desirable to expand the prosthesis, then collapse or partially collapse the prosthesis. Such capability is beneficial to the surgeon to properly position or re-position the prosthesis. In accordance with the present invention, the prosthesis may be self-expanding, or may be expandable using an inflatable device, such as a balloon or the like.

A delivery apparatus for the prosthesis may include an outer sheath, comprising an elongated tubular member having distal and proximal ends, and an inner shaft located coaxially within the outer sheath, the shaft having a distal end and a proximal end. The distal end of the shaft further including at least two grooves disposed thereon. The flanges of the first prosthesis are configured to releasably engage the grooves of a portion of the delivery device as is explained in detail subsequently.

An exemplary embodiment of a system for treating an abdominal aortic aneurysm according to the present invention is shown in Figure 1. For the purpose of this embodiment, the system is deployed in the infrarenal neck 101 of the abdominal aorta, upstream of where the artery splits into first and second common iliac arteries. Figure 1 shows the first prosthesis or stent gasket 10 positioned in the infrarenal neck 101; two second prostheses, 11a and 11b, the proximal ends of which matingly engage a proximal portion of stent gasket 10, and the distal ends of which extend into a common iliac artery 1 or 2. As illustrated, the body of the second prosthesis forms a conduit or fluid flow path that passes through the location of the aneurysm 100. In preferred embodiments of the invention, the components of the system define a fluid flow path that bypasses the section of the artery where the aneurysm is located.

As noted in more detail below in relation to specific system components, some prostheses of the present invention may be configured to seal and/or anchor the system in place, and/or to receive and position other prostheses. Typically these prostheses do not themselves define a fluid flow path. Other prostheses may be configured to define at least one fluid flow path. Typically, these prostheses define a channel or the like through which fluid, such as blood, flows. This channel or fluid flow path typically begins upstream of, or in an upstream portion of, a component of the system. In some embodiments of the invention, the fluid flow path bypasses the aneurysm.

These and other features of the prosthetic devices and systems of the present invention will be described in more detail below.

### FIRST PROSTHESIS OR SEALING PROSTHESIS

The first prosthesis includes a support matrix or stent that supports a sealing material or foam, at least a portion of which is positioned across a biological fluid flow path, e.g., across a blood flow path. In preferred embodiments of the invention, the first prosthesis, the stent, and the sealing material are radially expandable, and define a hollow space between a proximal portion of the prosthesis and a distal portion of the prosthesis. The first prosthesis may also include one or more structures for positioning and anchoring the prosthesis in the artery, and one or more structures for engaging and fixing at least one second prosthesis in place, e.g., a bypass prosthesis.

The support matrix or stent of the first prosthesis may be formed of a wide variety of materials, may be configured in a wide variety of shapes, and their shapes and uses are well known in the art. Exemplary prior art stents are disclosed in US-4733665 (Palmaz), US-4739762 (Palmaz) and US-4776337 (Palmaz).

In preferred embodiments of the invention, the stent of the first prosthesis is a collapsible, flexible, and self-expanding lattice or matrix formed from a metal or metal alloy, such as nitinol or stainless steel. Structures formed from stainless steel may be made self-expanding by configuring the stainless steel in a predetermined manner, for example, by twisting it into a braided configuration. More preferably, the stent is a tubular frame that supports a sealing material. The term tubular, as used herein, refers to any shape having a sidewall or sidewalls defining a hollow space or lumen extending therebetween; the cross-sectional shape may be generally cylindrical, elliptic, oval, rectangular, triangular, or any other shape. Furthermore, the shape may change or be deformable as a consequence of various forces that may press against the stent or prosthesis.

The sealing material or gasket member supported by the stent may be formed of a wide variety of materials, may be configured in a wide variety of shapes, and their shapes and uses are well known in the art. Exemplary materials for use with this aspect of the invention are disclosed in US-4739762 (Palmaz) and US-4776337 (Palmaz).

The sealing material or gasket member may comprise any suitable material. Exemplary materials are composed of a biodurable and biocompatible material, including but are not limited to, open cell foam materials and closed cell foam materials. Exemplary materials include polyurethane, polyethylene, polytetrafluoroethylene; and other various polymer materials, preferably woven or knitted, that provide a flexible structure, such as a polyester (such as that sold under the trade mark Dacron) Highly compressible foams are particularly preferred, preferably to keep the crimped profile low for better delivery. The sealing material or foam is preferably substantially impervious to blood when in a compressed state.

The sealing material may cover one or more surfaces of the stent i.e., can be located along an interior or exterior wall, or both, and extends across the proximal end or a proximal portion of the stent. The sealing material helps impede any blood trying to flow around the first prosthesis, e.g., between the first prosthesis and the arterial wall, and around one or more bypass prostheses after they have been deployed within the lumen of the first prosthesis (described in more detail below).

In preferred embodiments of the invention, the sealing material stretches or covers a portion of the proximal end of the stent and along at least a portion of the outside wall of the stent.

In some embodiments of the invention, it may be desirable for the portion of the sealing material covering the proximal portion of the stent to include one or more holes, apertures, points, slits, sleeves, flaps, weakened spots, guides, or the like for positioning a guidewire, for positioning a system component, such as a second prosthesis, and/or for engaging, preferably matingly engaging, one or more system components, such as a second prosthesis. For example, a sealing material configured as a cover or the like, and having a hole, may partially occlude the stent lumen.

These openings may be variously configured, primarily to conform to its use. These structures promote proper side by side placement of one or more, preferably multiple, prostheses within the first prosthesis, and, in some embodiments of the invention, the sealing material may be configured or adapted to assist in maintaining a certain shape of the fully deployed system or component. Further, these openings may exist prior to deployment of the prosthesis, or may be formed in the prosthesis as part of a deployment procedure. The various functions of the openings will be evident from the description below. In exemplary embodiments of the invention, the sealing material is a foam cover that has a single hole.

The sealing material may be attached to the stent by any of a variety of connectors, including a plurality of conventional sutures of polyvinylidene fluoride, polypropylene, polyester (such as that sold under the trade mark Dacron), or any other suitable material and attached thereto. Other methods of attaching the sealing material to the stent include adhesives, ultrasonic welding, mechanical interference fit and staples.

One or more markers may be optionally disposed in or on the stent between the proximal end and the distal end. Preferably, two or more markers are sized and/or positioned to identify a location on the prosthesis, or to identify the position of the prosthesis, or a portion thereof, in relation to an anatomical feature or another system component.

First prosthesis is typically deployed in an arterial passageway upstream of an aneurysm, and functions to open and/or expand the artery, to properly position and anchor the various components of the system, and, in combination with other components, seal the system or portions thereof from fluid leaks. For example, the first prosthesis may be deployed within the infrarenal neck, between an abdominal aortic aneurysm and the renal arteries of a patient, to assist in repairing an abdominal aortic aneurysm.

Figures 1-3 show an exemplary sealing prosthesis of the present invention. Sealing prosthesis 10 includes a cylindrical or oval self-expanding lattice, support, or stent 12, typically made from a plurality of interconnected struts 13. Stent 12 defines an interior space or lumen 18 having two open ends, a proximal end 14 and a distal end 16. One or more markers 15 may be optionally disposed in or on the stent between the proximal end 14 and the distal end 16.

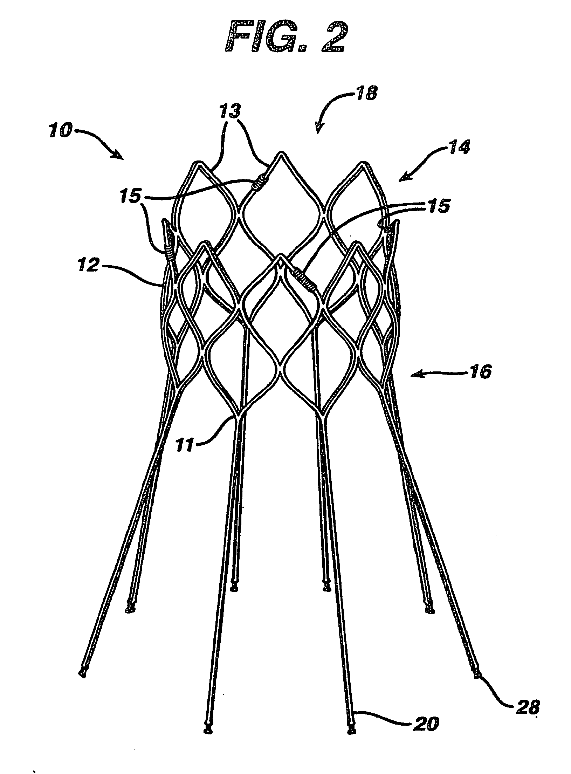

Stent 12 may further include at least two, but preferably eight (as shown in Figure 2), spaced apart longitudinal legs 20. Preferably, there is a leg extending from each apex 11 of diamonds formed by struts 13. At least one leg, but preferably each leg, includes a flange 28 adjacent its distal end which, as is described in greater detail below, allows for the stent 12 to be retrievable into its delivery apparatus after partial or nearly full deployment thereof so that it can be turned, or otherwise repositioned for proper alignment.

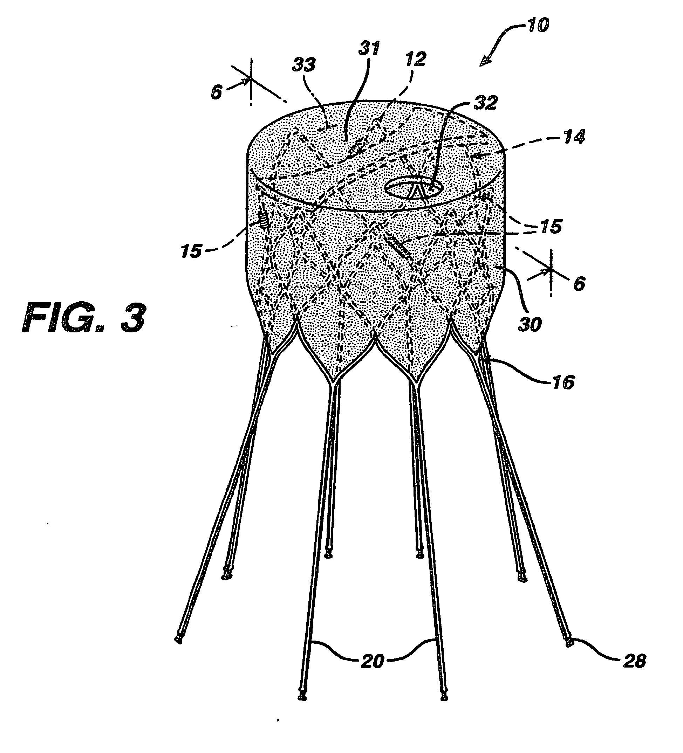

Figure 3 shows the gasket or sealing material 30 covering the proximal end of stent gasket 10. In the exemplary embodiment shown in Figure 3, sealing prosthesis 10 includes a sealing material 30 having a first opening or hole 32 and a second opening or slit 33. The gasket material covers at least a portion of the interior or exterior of the stent, and most preferably covers substantially all of the exterior of the stent. For example, gasket material 30 may be configured to cover stent 12 from the proximal end 14 to the distal end 16, but preferably not covering longitudinal legs 20.

The sealing material helps impede any blood trying to flow around bypass prostheses 11a and 11b after they have been deployed (as shown in Figure 1), and from flowing around the precursor stent itself. For this embodiment, sealing material 30 is a compressible member or gasket located along the exterior of the stent 12 and at least a portion of the interior of the stent 12.

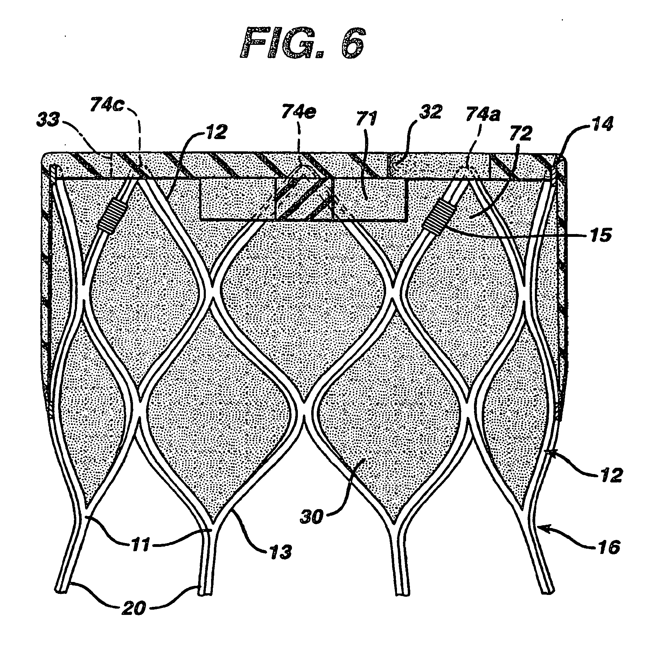

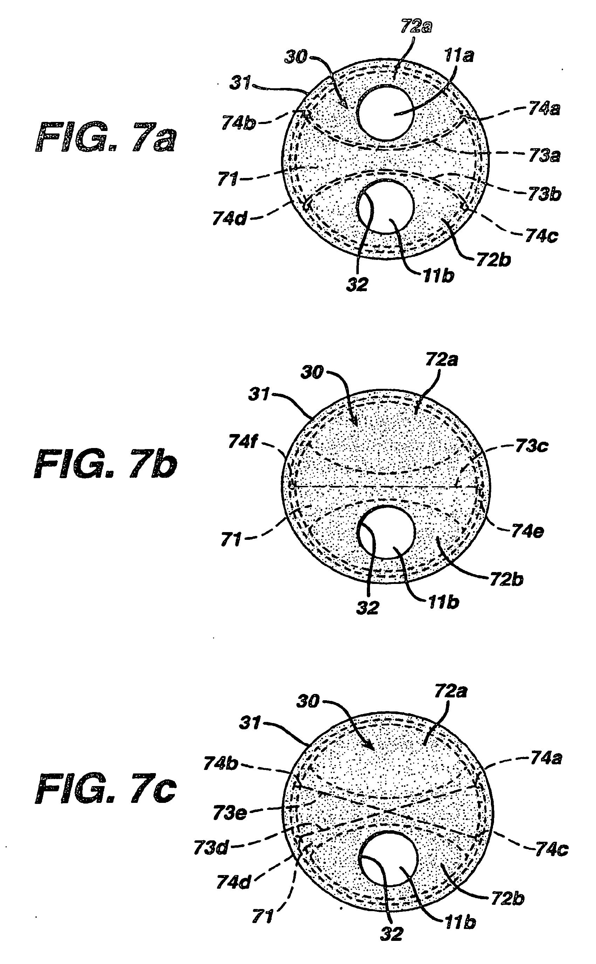

Preferred embodiments of the invention are illustrated in Figures 6 and 7a to 7e. These Figures show a first prosthesis 10 having a gasket material 30 that covers at least a portion of the proximal end of the first prosthesis 10. The gasket material 30 includes a partition that extends approximately across the diameter of the cross section of the first prosthesis 10, wherein the partition includes a thicker gasket material, or further includes a foam or the like. The partition may be formed from any of the gasket or foam materials described above.

The exemplary embodiments illustrated in Figures 6 and 7a to 7e include a thicker partition 71 in roughly an hourglass shape, although other shapes and sizes may be used. The partition defines at least one section 72 within the prosthesis having less material or the like, these sections being configured for receiving a proximal end of a second prosthesis, as is described in more detail below. In the exemplary embodiments shown in Figures 7a to 7e, partition 71 defines a first section 72a and a second section 72b; first section 72a is configured to receive a first second prosthesis 11a, and second section 72b is configured to receive a second second prosthesis 11b, as described below.

In accordance with the present invention, it may be desirable to include one or more fibres, threads, filaments, straps, or the like for further defining a section 72. In the description below, the word fibre will be used as a shorthand descriptor for the element that includes fibres, threads, filaments, straps, or the like. In preferred embodiments of the invention, the fibre, etc., assists in positioning a second prosthesis 11a,b.

In accordance with the present invention, the fibre may be formed from any material and/or comprise any construction suitable for use in a biological environment, e.g., suitable for use in a blood vessel. The fibre may be woven or non-woven, formed of a synthetic or natural material, and/or single or multi- filament. Exemplary materials for forming the fibre include but are not limited to polyester, polyester (such as that sold under the trade mark Dacron), polytetrafluoroethylene (such as that sold under the trade mark Teflon), polyurethane, porous polyurethane, silicone, polyethylene terephthalate, polyvinylidene fluoride, expanded polytetrafluoroethylene (ePTFE). The fibre or thread may also take on other forms, for example, the fibre or thread may be formed from glues or adhesives or by melting sections of the gasket material. In addition, the fibre or thread may comprise struts deformed out of the circumferential plane.

The end or ends of the fibre may be unattached or attached. In a preferred embodiment of the invention, both ends of the fibre are attached or fixed. For example, the ends may be sewn or fixed to the cover 31. In a preferred embodiment of the invention, the ends of the fibre are fixed to a strut 13, even more preferably to a proximal portion of stent 12. One or more ends of the fibre may be fixed to the stent 12 or the strut 13 by threading, knotting, sewing, with adhesives, or any other mechanism for fixing the end of the fibre in place.

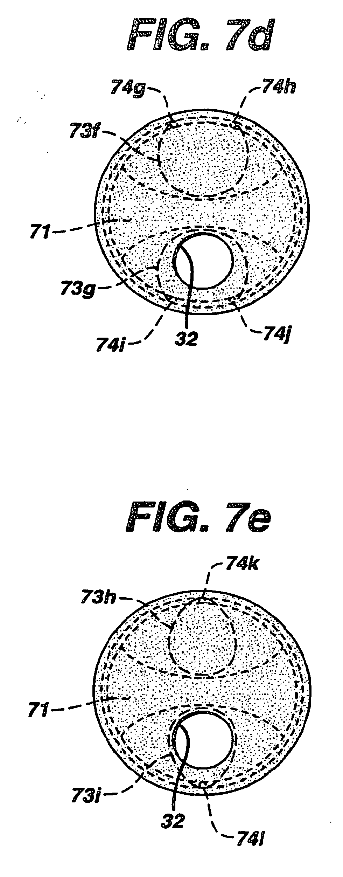

In the exemplary embodiments of the invention illustrated in Figures 7(a-e), fibre 73 may be variously configured. In Figure 7a, fibres 73a and 73b may be interwoven in the cover 31, and define or form first section 72a and a second section 72b, as noted above. As shown, the ends of the fibres may be fixed to a strut; see 74a, 74b, 74c, and 74d. In Figure 7b, a single fibre 73c may be positioned across the diameter of the cover 31, and is fixed to a strut at 74e and 74f. In Figure 7c, one or more crossed fibres 73d and 73e may be used to form or define sections 72a and 72b respectively. In the illustrated embodiments, the ends may be attached to the stent 12 at 74a, 74b, 74c, and 74d. In Figure 7d, fibres 73f and 73g may be positioned such that they substantially conform to the hourglass shape of the thicker partition 71. As illustrated, the fibres 73f, 73g form a substantially oval pattern and are attached to the stent 12 at 74g, 74h, 74i and 74j. In Figure 7e, fibres 73h and 73i may be positioned such that they form substantially circular configurations around the second prostheses 11a, b. As illustrated, the fibres 73h, I may be attached to the stent 12 at 74k and 1.

In some embodiments according to the present invention, it may be desirable to use a fibre that is frangible or breakable. In these exemplary embodiments of the invention, the fibre breaks as the unexpanded prosthesis is expanded to its fully deployed position. Alternatively, the ends of the fibres may be releasably fixed to the stent or strut when the prosthesis is in a collapsed condition, with one or more ends releasing as the prosthesis expands to its fully deployed position.

These structures promote proper side by side placement of one or more, preferably multiple, prostheses within the first prosthesis 10.

### SECOND PROSTHESIS

The second prosthesis is a bypass conduit or the like that is typically deployed in an arterial passageway upstream of an aneurysm, and establishes a fluid flow path through the system or a portion thereof. In some embodiments of the invention, the second prosthesis defines a fluid flow path that passes through the arterial segment having the aneurysm, e.g., bypassing the aneurysm. In these embodiments of the invention, the second prosthesis extends from a healthy portion of the artery, through the arterial segment having the aneurysm, and into another healthy portion of the artery or another artery. In some embodiments of the invention, the second prosthesis defines a fluid flow path from one portion of the system, e.g., a proximal portion or end, to another portion, e.g., a distal portion or end, or an intermediate portion.

The second prosthesis functions to bypass the portion of the conduit containing the aneurysm, and to properly position and/or anchor the proximal end of the system in an artery .The second prosthesis may also include one or more structures for positioning and anchoring the second prosthesis in the artery or in the first prosthesis. In a preferred embodiment of the invention, the second prosthesis is adapted to engage the first prosthesis.

One or more markers may be optionally disposed in or on the stent between the proximal end and the distal end. Preferably, two or more markers are sized and/or positioned to identify a location on the prosthesis, or to identify the position of the prosthesis, or a portion thereof, in relation to an anatomical feature or another system component. In preferred embodiments of the invention, fluoroscopically identifiable sutures or staples are used; these sutures or staples may also attach the graft material to the stent.

The second prosthesis typically includes a support matrix or stent that supports a graft material. One end of the second prosthesis is typically adapted to engage one or more portions of a first prosthesis. In preferred embodiments of the invention, the proximal end of second prosthesis is adapted to matingly engage a proximal portion of the first prosthesis. The second prosthesis may optionally include at least one attachment structure on its distal end for engaging and securing the prosthesis in a portion of an artery downstream of the aneurysm.

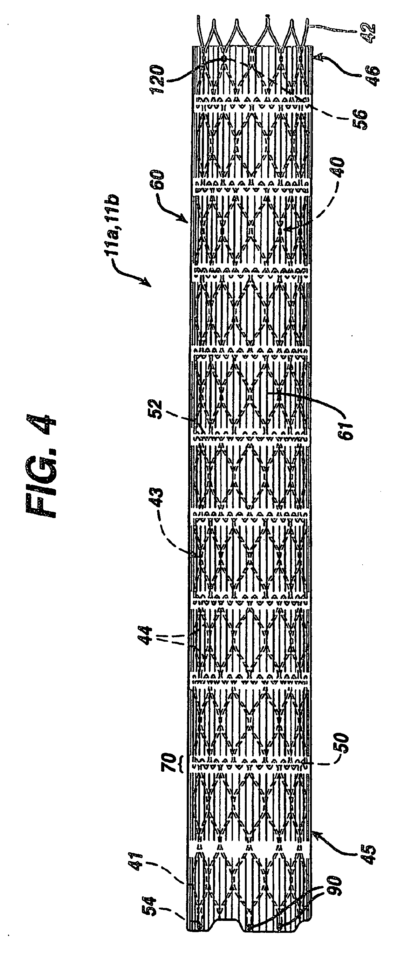

Figures 1 and 4 show an exemplary second or bypass prosthesis 11a, b of the present invention. Second prosthesis 11a,b includes a substantially cylindrical self-expanding lattice, support, or stent 40, typically made from a plurality of interconnected struts 44. Lattice 40 defines an interior space having two open ends, a proximal end 41 and a distal end 42. The interior and/or exterior surfaces of lattice 40 may be covered by or support at least one graft material 60. These and other features of the second prosthesis will be described in more detail below.

### THIRD PROSTHESIS

A third prosthesis is a second prosthesis that does not pass through the aneurysm. The third prosthesis is a bypass conduit or the like that is typically deployed in an arterial passageway upstream of an aneurysm, and extends from a healthy portion of a first artery into another healthy portion of the first artery or into a second artery. The third prosthesis functions to establish a fluid flow path or channel from an upstream portion of the system into an artery upstream of the aneurysm, for example, the renal arteries 3, 4 illustrated in Figure 1, and to properly position and/or anchor a proximal end of the system in an artery. The third prosthesis may also include one or more structures for positioning and anchoring the third prosthesis in the artery or in the first prosthesis. Any third prosthesis may be configured as described above for any second prosthesis.

In a preferred embodiment of the invention, the third prosthesis is adapted to engage the first prosthesis.

### STENT

Any of the stents of the present invention form a support or lattice structure suitable for supporting a graft material. In preferred embodiments of the invention, the stent defines a channel through which a fluid, such as blood, may flow. A typical stent comprises an expandable lattice or network of interconnected struts. In preferred embodiments of the invention, the lattice is fabricated, e.g., laser cut, from an integral tube of material.

In accordance with the present invention, the stent may be variously configured. For example, the stent may be configured with struts or the like that form repeating geometric shapes. One skilled in the art will readily recognize that a stent may be configured or adapted to include certain features and/or to perform a certain function(s), and that alternate designs may be used to promote that feature or function.

In some exemplary embodiments of the invention, the struts of the stent gasket form a matrix having diamond shapes. In the embodiment of the invention shown in Figure 2, the matrix or struts of stent 12 are configured into diamond shapes, preferably having approximately eight diamonds. In a most preferred embodiment of the invention, the fully expanded diamond pattern of a first prosthesis has angles of about forty-five to fifty-five degrees at their distal and proximal ends.

In the exemplary embodiment of the invention shown in Figure 4, the matrix or struts of stent 40 may be configured into at least two hoops 43, each hoop 43 comprising a number of struts 44 having a diamond shape, having approximately nine diamonds. A second and/or third prosthesis, such as second prosthesis 11a,b, may further include a zig-zag shaped ring 50 for connecting adjacent hoops to one another. The zig-zag shaped rings may be formed from a number of alternating struts 52, wherein each ring has fifty-four struts.

The diamond pattern for the anchors, as well as the other hoops, provide the hoops with radial and longitudinal stiffness. The longitudinal strength provides for better mechanical fixation of stent 40 to a graft material (described below). The radial strength provides the proximal hoop 45 with better attachment and sealing to the graft material, and provides the distal hoop 46 with better fixation and sealing to the arterial wall. Further, the distal hoop may be flared, and may be exposed after the graft material has been attached to the stent.

In one preferred embodiment, the proximal and distal hoops have greater radial and longitudinal strength than the hoops therebetween. This creates a stent graft having stiff ends for anchoring, but a more flexible body for navigation through the vasculature. The stiffer ends may be accomplished by changing the dimensions of the struts for the end hoops, or by varying the heat treatment of the end hoops during manufacture. The rings allow the stent to bend more easily, and generally provide for more flexibility when the stent is being delivered through a tortuous vessel. When a non-compliant graft is attached to a stent, the strength of the diamond hoops scaffolds any graft folding into the blood flow lumen, while maintaining a tight kink radius.

In accordance with some embodiments of the present invention, the proximal and/or distal end of a stent may include one or more anchors and/or one or more struts of the stent configured into an anchor. One or more anchors, commonly referred to as recapture legs, may also be configured to releasably engage a delivery device, such as a catheter, or a portion thereof.

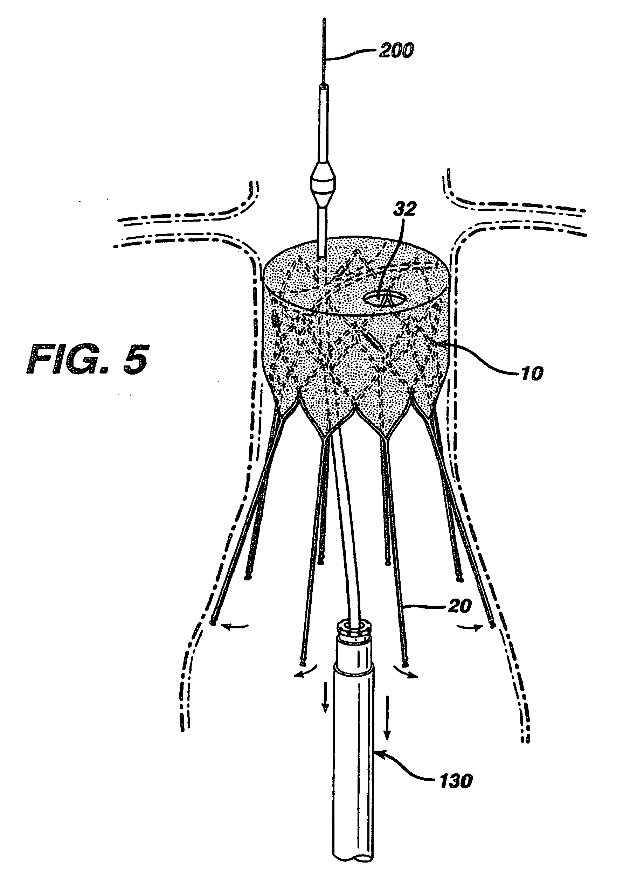

The distal end of the stent is preferably configured to engage a complementary structure on a delivery device, such as a catheter or a portion thereof. For example, the distal end of the stent may include one or more keys or flanges that engage, preferably releasably engage, a corresponding latch on the catheter. An exemplary configuration is shown in Figure 5. It is intended that the invention should not be limited by the precise structures used to engage the stent to the delivery device.

In the embodiments of the invention shown in Figures 1-3, the stent may include one or more anchors keys or flanges 28 configured to engage a corresponding structure on a delivery device 130 (shown in Figure 5). In accordance with the present invention, the delivery apparatus may include a collar having one or more grooves or the like adapted to releasably engage one or more complementary structures on a stent or prosthesis of the present invention. Such an anchor/delivery device configuration is particularly suited to partially deploying a prosthesis of the present invention, and to position or re-position the prosthesis.

Any of the stents of the present invention may be formed of any material suitable for functioning *in vivo* as a support for graft material. A stent of the present invention may be formed of a wide variety of materials, all of which are well known to those skilled in the art. In some embodiments of the present invention, the stent is formed from a metal or metal alloy. In preferred embodiments of the present invention, the stent is formed from superelastic Nickel Titanium alloys (Nitinol). Descriptions of medical devices which use such alloys can be found in US-4665906 and EP-A-928606. A stent according to the present invention is preferably laser cut from a tubular piece of nitinol and thereafter treated so as to exhibit shape memory properties at body temperature. In preferred embodiments of the invention, the stent material is expandable or collapsible, i.e., moveable from a first closed position to a second open position, or vice versa.

### GRAFT MATERIAL

An inner or outer surface of a stent of the present invention may be covered by or support a graft material. Graft material 60 can be made from any number of materials known to those skilled in the art, including woven polyester, polyester (such as that sold under the trade mark Dacron), polytetrafluoroethylene (such as that sold under the trade mark Teflon), polyurethane, porous polyurethane, silicone, polyethylene terephthalate, expanded polytetrafluoroethylene (ePTFE) and blends of various materials.

In some embodiments of the invention, it may be desirable to incorporate a biodegradable, or degradable material, such as albumin, collagen, or any type of collagen. A graft material that is biodegradable would erode or dissolve over time; it is believed that the eroding graft material may be replaced by one or more biofusion constituents.

In some embodiments of the present invention, it may be desirable to incorporate a biodegradable, or degradable material, such as albumin, collagen, or a collagen. A graft material that is biodegradable would erode or dissolve over time; however, it is believed that a layer of endothelium may grow as the graft material erodes. It is further believed that these new layers of endothelium may provide a new, fluid impervious lining within the aneurysm.

It is preferred that all of the foregoing materials be porous to allow for an intimal layer to form a biofusion structure or matrix.

The graft material may be variously configured, preferably to achieve predetermined mechanical properties. For example, the graft material may incorporate a single or multiple weaving and/or pleating patterns, or may be pleated or unpleated. For example, the graft may be configured into a plain weave, a satin weave, include longitudinal pleats, interrupted pleats, annular or helical pleats, radially oriented pleats, or combinations thereof. Alternately, the graft material may be knitted or braided. In the embodiments of the invention in which the graft material is pleated, the pleats may be continuous or discontinuous. Also, the pleats may be oriented longitudinally, circumferentially, or combinations thereof.

As shown in Figure 4, graft material 60 may include a plurality of longitudinal pleats 61 extending along its surface, generally parallel to the longitudinal axis of the prosthesis. The pleats allow the prosthesis to collapse around its centre, much as it would be when it is delivered into a patient. This provides a relatively low profile delivery system, and provides for a controlled and consistent deployment therefrom. It is believed that this configuration minimizes wrinkling and other geometric irregularities. Upon subsequent expansion, the prosthesis assumes its natural cylindrical shape, and the pleats or folds uniformly and symmetrically open.

In addition, pleats 61 help facilitate stent graft manufacture, in that they indicate the direction parallel to the longitudinal axis, allowing stent to graft attachment along these lines, and thereby inhibiting accidental twisting of the graft relative to the stent after attachment. The force required to push the stent-graft out of the delivery system may also be reduced, in that only the pleated edges of the graft make frictional contact with the inner surface of the delivery system. One further advantage of the pleats is that blood tends to coagulate generally uniformly in the troughs of the pleats, discouraging asymmetric or large clot formation on the graft surface, thereby reducing embolus risk.

As shown in Figure 4, the graft material may also include one or more, and preferably a plurality of, radially oriented pleat interruptions 70. The pleat interruptions are typically substantially circular and are oriented perpendicular to longitudinal axis. Pleat interruptions 70 allow the graft and prosthesis to bend better at selective points. This design provides for a graft material that has good crimpability and improved kink resistance.

The graft material as described above is preferably highly compressible, which also promotes a low crimped profile for better delivery characteristics.

In accordance with the present invention, the graft material may be impervious or substantially impervious to the flow of blood, or may be porous.

A graft material is impervious if it prevents blood from passing through the graft material on contact with blood or after the graft material is saturated with blood. Choice of the flow characteristics of a graft material are well known to those skilled in the art, and are tied in part to the intended function of the prosthesis or portion of the prosthesis. For example, it may be desirable for the graft material that forms the cover of the first prosthesis to be impervious or substantially impervious to the flow of blood. Alternately, it may be desirable for a graft material to be porous or partially porous to promote biofusion.

The foregoing graft materials may be knitted or woven, and may be warp or weft knitted. If the material is warp knitted, it may be provided with a velour, or towel like surface; which is believed to speed the formation of blood clots, thereby promoting the integration of a prosthesis or prosthesis component into the surrounding cellular structure.

A graft material may be attached to a stent or to another graft material by any number of structures or methods known to those skilled in the art, including adhesives, such as polyurethane glue; a plurality of conventional sutures of polyvinylidene fluoride, polypropylene, polyester, or any other suitable material; ultrasonic welding; mechanical interference fit; and staples.

As stated above, a stent preferably has a graft member attached thereto. The graft member covers at least a portion of the interior or exterior of the stent, and most preferably covers substantially all of the exterior of the stent. In some embodiments of the invention, second prosthesis 11a, b includes graft material 60 that covers only a portion of the distal end 42 of matrix 40. See, for example, Figure 4.

In an alternate design, graft material may not be utilized on either end of the stent. For example, on any endolegs, prostheses, extension cuffs, stent gaskets or other covered stents, both ends thereof may be left uncovered. The body has the ability to cover the exposed portions of the stent with endothelial cells and thus these exposed portions become endothelialised or incorporated into the vessel wall. This may be an important factor in the long term stability of the system. Essentially, over long periods of time, the aneurysmal sac can and will shrink if it is totally excluded from blood flow. This shrinkage changes the morphology of the aortic region that has been treated with the bypass prostheses. If all ends of the system are firmly anchored in the arterial vessel, as is the case when the ends are covered with endothelium cells, the system will be better able to withstand these morphological changes.

In accordance with the present invention, it may be highly desirable to provide a graft material that limits or eliminates the amount of blood that passes between the graft and the arterial wall, to provide a catheter-delivered graft or prosthesis that extends through a longer portion of an artery, to improve the anchoring mechanisms between two prostheses, to improve the anchoring mechanism between the prosthesis and the arterial wall or an interluminal cavity within an artery, and to improve the fluid dynamic and performance characteristics of the implanted prosthesis.

In preferred embodiments of the invention, on the downstream end of a prosthesis that provides a fluid flow path, the graft material may be shaped to conform to the pattern of struts supporting the graft material. For example, the downstream edge of graft material 60 is preferably fully supported by the underlying stent. Such a configuration may be desirable to prevent unsupported sections of the graft material from folding into the lumen of the stent.

### MARKER

As noted above, a stent and/or prosthesis of the present invention may include one or more markers. One skilled in the art will recognize that one or more markers may be positioned on the stent, the graft material, or on the prosthesis. In preferred embodiments of the invention, the marker are used to identify the position of the stent or prosthesis in relation to a body part and/or in relation to another stent or prosthesis, and/or to identify the position of one part of the prosthesis relative to another part. In most preferred embodiments of the invention, the marker(s) is/are used to identify a position *in vivo.*

As shown in Figures 2 and 3, a stent, such as stent 12 and/or 40, preferably includes one or more radiopaque markers 15. Exemplary materials for forming markers include but are not limited to tantalum, platinum, iridium, and gold. As shown, markers 15 are coils of radiopaque metal, wrapped around the struts of the stent. Markers 15 are preferably made from 0.19 mm (0.0075 inch) diameter tantalum (Ta) wire wrapped tightly around the struts.

The number, location, and size of the markers may vary, and the markers may be used alone or in combination to identify the position of a particular portion of the prosthesis. For example, a proximal marker adjacent aperture 32 may be five mm long and the proximal marker adjacent hole 33 may be two mm long. Also, two distal markers may be one hundred eighty degrees apart, and a proximal marker may be positioned equidistant from each of the distal markers. In this exemplary configuration; the proximal marker then aids proper rotational positioning of the device.

### CONNECTORS

Some exemplary embodiments of a prosthesis according to the present invention may include one or more connectors. In some embodiments of the invention, the connectors are used to engage or connect one prosthesis or component to another. In some embodiments of the invention, the connectors may be used to attach the gasket material or graft material to a stent or lattice.

As noted above, one skilled in the art will recognize that a variety of materials and methodologies may be used to connect one prosthesis to another, or to attach the graft material to a stent. Exemplary connectors include but are not limited to sutures, staples, rivets, or the like. In preferred embodiments of the invention, the connector is a suture or staple, even more preferably, having a knotted or nub end. Further, a connector may be formed from a radiopaque material or a fluorescent material, each of which allow the connector to be used as a marker.

In accordance with the present invention, it may be desirable to incorporate in a prosthesis a connector adapted for use with a lattice-like stent. As illustrated in Figure 4, a first connector 54 may be configured for use at an end portion of a stent, preferably at an end portion of a strut 44. A second connector 56 may be configured for use at an internal portion of a stent, preferably at the junction between two struts 44.

Alternately, a connector assembly for receiving a rivet, staple, suture, or the like, may include two apertures, each aperture configured to receive a leg of the rivet, staple, suture, or the like. In this exemplary embodiment of the present invention, the end of each leg is preferably formed into a knot, nub, or spherical end that is of larger diameter than the diameter of the aperture. Preferably, all of the elements noted above are assembled, the legs are passed through the apertures, and the end of each leg is formed into a nub. Alternately, one end may be formed into a nub prior to placement through the aperture, with the second end being formed into a nub after assembly of all the elements.

The structures and functions of the second connector 56 are similar or the same as those described above for the first connector.

The number of connectors and staples are typically dictated by the size and structure of a particular stent. For example, there may be six first connectors and three-second connectors.

The above staple aperture design or connector assembly has many advantages for attaching gasket material or a graft material to a stent. Because the legs of the staple are folded around and embedded within a pocket or the like, any risk of puncturing an inflation balloon is minimized. In addition, the structural integrity of the prosthesis is increased because staples more securely attach the graft material to the stent, as compared to prior art designs which use sutures or adhesives to attach the graft to the stent.

Staples 90 and 120 as illustrated in Figure 4 may be made from any number of materials known in the art, including tantalum alloys, platinum alloys or stainless steel, such as a grade of type 316 stainless steel. The staples may take on other configurations and shapes, and may be coated for lubricity purposes, wear resistance and for the prevention of corrosion. Essentially, the coating may be used for increased durability. The staples may be formed from a radiopaque material to identify the location of the staple, and to act as a marker to identify the location of a portion of the prosthesis. Using a different number of radiopaque staples on a distal end of a stent as compared to a proximal end further assists in identifying the position of the prosthesis.

### METHODS

The prosthesis of the invention can be used in a method which includes delivering and positioning a system or component of a system in a fluid conduit, such as an aorta. The components described above permit intraluminal delivery into an aorta. This is accomplished by percutaneously inserting the prostheses into the same or different arteries, e.g., an iliac artery, and navigating them to the site of the aneurysm. This type of procedure is similar to delivery of angioplasty catheters and guiding catheters into the human vasculature. Upon proper positioning, the system components may be deployed either through a radially, outwardly extending force, e.g., expanding a balloon, or, if a self-expanding stent, by releasing the stent anchors from a constraint. Once fully deployed: at least one passageway is formed bypassing the aneurysm. As shown in Figure 1, it may be desirable to form two fluid flow paths bypassing the aneurysm, each fluid flow path extending into a separate downstream artery.

In preferred embodiments of the invention, the first prosthesis is a stent gasket, even more preferably, a stent gasket that expands automatically against the wall of the artery. As the stent gasket expands, proximal longitudinal legs allow the stent gasket diamond rings to expand, thereby anchoring the stent in place. The method also includes delivering and positioning at least one-second prosthesis. In preferred embodiments of the invention, the second prosthesis is a bypass conduit for extending through an aneurysm. The second prosthesis is typically positioned within the first prosthesis, preferably into and through a hole in the first prosthesis cover. In most preferred embodiments of the invention, the hole is slightly smaller in diameter than the expanded diameter of the second prosthesis, thus sealingly engaging the second prosthesis in the first prosthesis. The sealed configuration of the second prosthesis within the first prosthesis forms a fluid pathway through the assembly or system, thereby bypassing the aneurysm.

Figures 1 and 5 generally show how the system of the present invention may be deployed *in vivo.* One skilled in the art will readily recognize that typical delivery device, such as a catheter, includes a guidewire 200 or the like that passes through an aperture in the cover of the first prosthesis, and a collar or the like that releasably engages at least one anchor on the prosthesis. Once the anchors are released from the collar, the first prosthesis can expand, preferably automatically The portion of the delivery device containing the collar can then be removed from the artery, typically leaving the guidewire in place, still positioned in an aperture of the first prosthesis cover. The guidewire can then be used to guide another prosthesis or prostheses, such as a second prosthesis, into position

As an example, the collar of the delivery device engaged to the prosthesis, may be positioned within a sheath or the like until the prosthesis is delivered. In preferred embodiments of the invention, a portion of the prosthesis may be partially deployed and/or positioned. Once it is determined that the prosthesis is in its proper position, the collar can be pushed out of the sheath, thereby releasing the anchors from the collar. If the prosthesis is a self-expanding prosthesis, release of the flanges will allow the prosthesis to deploy automatically If the prosthesis is not self-expanding, a deflated balloon or the like may be delivered to the interior of the prosthesis using the guidewire. When the balloon is inflated, it will expand the prosthesis into its fully deployed position, i.e., fully expanded radially

As is evident to one skilled in the art, precisely placing a component of the system may be critical. The interventionist must have precise placemen the components to ensure adequate repair of the aneurysm. The present invention allows the interventionist to fully deploy a component within the body without fully releasing the entire component from the delivery device. The anchors releasably interlock with complementary structures, such as grooves, on the delivery device, and, if the interventionist decides that the placement of the component is incorrect, the outer member of the delivery device may be moved relative to an inner member, thereby resulting in the prosthesis being retrieved or retracted within the delivery device. The extended legs and anchors allow the interventionist to temporarily position the prosthesis before full deployment. Once the interventionist is satisfied with a prosthesis' position, the legs 20 may be released from their engagement with the delivery device

In order to prevent the physician from prematurely completely deploying a prosthesis, a releasably stop may be preferably placed on the delivery device.

In preferred embodiments of the invention, the system is used to bypass an abdominal aortic aneurysm (AAA) A method for treating or bypassing an AAA includes delivering, preferably percutaneously, a first prosthesis or precursor stent, or one of its components (e.g., the gasket member may be delivered separately, if desired). The components of the system are typically delivered through one of the iliac arteries and deployed within the infrarenal neck, between an abdominal aortic aneurysm and the renal arteries of a patient. Once the first prosthesis is properly positioned or re-positioned, the legs and anchors are fully released from the delivery device The delivery device for the precursor stent may then be removed, without removing guidewire, and another guidewire may be inserted through the other iliac artery and into the first the prosthesis. If the second guidewire is on the wrong side of the interior of the first prosthesis, it will contact the occlusive member be prevented from easily advancing. The physician may then properly reposition the guide wire through hole 32.

Thereafter each delivery apparatus, each containing a sheathed second prosthesis, is inserted into iliac arteries 1 and 2 by sliding them over the guide wires; each of the two second prostheses are then positioned in the first prosthesis Thereafter, the second prostheses may be either separately or simultaneously deployed.

After proper delivery, first prosthesis 10 and second prostheses 11a and 11b should appear as they do in Figure 1. First prosthesis 10 along with its attached gasket material is firmly secured within an arterial section upstream of an aneurysm, and may or may not extend into one or more arteries.

In accordance with the present invention, a system for bypassing an aneurysm may establish one, and possible multiple, fluid flow paths through the system. When the system is placed in an artery upstream of a junction with one or more other arteries, the system permits fluid, such as blood, to flow through the proximal end of the system, and a portion of the blood may flow out of the system into one of the cross arteries. Another portion of the fluid will continue within the system, bypassing the aneurysm and out of the system into one or more downstream arteries. A method therefore includes establishing one or more fluid flow paths. In an example, the method includes establishing a first fluid flow path through the system, wherein the first fluid flow path bypasses the aneurysm. The method may further include establishing at least one-second fluid flow path, wherein the second fluid flow path passes through a portion of the system, and passes out of an intermediate portion of the system into an artery or arteries

It is important to note that even though self-expanding stents are utilized, balloons may be utilized for tacking them into position if necessary.

## Claims

1. A prosthesis for sealing an aneurysm repair assembly comprising a stent and a gasket material engaging at least a portion of said stent wherein a portion of said gasket material is positioned at the proximal end and across the diameter of the cross section of said prosthesis, **characterised in that**;
said portion of said gasket material comprises a partition (71) and a thread (73a, 73b, 73c, 73d, 73e, 73f, 73g, 73h, 73i) defining a section (72) having less material than the partition (71).

2. The prosthesis of claim 1 wherein said thread is configured across said proximal end to engage a proximal end of a second prosthesis.

3. A system for repairing an aneurysm comprising:
a first prosthesis (10) according to claim 2; and
at least one second prosthesis (11a, 11b).

4. The system of claim 3 wherein said first thread positions said second prosthesis (11a, 11b) within said first prosthesis (10).

5. The system of claim 3 wherein said first thread further defines a second section configured to engage another second prosthesis (11a, 11b).

6. The system of claim 3 further comprising a second thread defining a second section.

7. The system of claim 6 wherein said second section is configured to engage a second prosthesis (11a, 11b).

8. The system of claim 3 further comprising a third thread defining a third section.

9. The system of claim 8 wherein said third section is configured to engage a third prosthesis.

10. The system of claim 3 further comprising a fourth thread defining a fourth section.

11. The system of claim 10 wherein said fourth section is configured to engage a fourth prosthesis.

12. The system of claim 3 wherein said first thread releasably engages said second prosthesis (11a, 11b).

13. The system of claim 12 wherein the first prosthesis (10) is moveable between an unexpanded condition and an expanded condition, and said thread engages said second prosthesis when the first prosthesis is in an unexpanded condition.

14. The system of claim 3, wherein the thread comprises struts deformed out of the circumferential plane of the stent.

15. A system for bypassing an aneurysm comprising a first prosthesis according to claim 2 and a second prosthesis, wherein said first prosthesis is adapted to engage a portion of an artery upstream of said aneurysm; wherein said second prosthesis is configured to provide a fluid flow path that bypasses said aneurysm, and wherein a proximal end of said second prosthesis is adapted to releasably engage said thread.

16. A system for bypassing an aneurysm comprising a first prosthesis according to claim 2 and a second prosthesis, wherein said first prosthesis comprises a matrix of interconnected struts and said gasket material covers at least a portion of said matrix; and wherein said second prosthesis comprises a matrix of interconnected struts and a graft material covering at least a portion of said matrix; wherein said first prosthesis is adapted to engage a portion of an artery upstream of said aneurysm, wherein said second prosthesis is configured to provide a fluid flow path that bypasses said aneurysm, and wherein a proximal end of said second prosthesis is adapted to releasably engage said thread.

17. The system of claim 15 wherein said second prosthesis further comprises a distal end configured to engage a portion of an artery downstream of said aneurysm.

## Patentansprüche

1. Prothese zum Abdichten eines Aneurysma-Reparaturaufbaus, die einen Stent und ein Dichtmaterial umfasst, das an wenigstens einem Teil des Stents angreift, wobei ein Teil des Dichtmaterials an dem proximalen Ende und quer über den Durchmesser des Querschnitts der Prothese angeordnet ist, **dadurch gekennzeichnet, dass** der Teil des Dichtmaterials eine Trennwand (71) und einen Faden (73a, 73b, 73c, 73d, 73e, 73f, 73g, 73h, 73i), der einen Abschnitt (72) mit weniger Material als die Trennwand (71) definiert, aufweist.

2. Prothese nach Anspruch 1, bei welcher der Faden quer über das proximale Ende angeordnet ist, um an einem proximalen Ende einer zweiten Prothese anzugreifen.

3. System zum Reparieren eines Aneurysma, das Folgendes umfasst:
eine erste Prothese (10) nach Anspruch 2; und
wenigstens eine zweite Prothese (11a, 11b).

4. System nach Anspruch 3, bei welchem der erste Faden die zweite Prothese (11a, 11b) in der ersten Prothese (10) positioniert.

5. System nach Anspruch 3, bei welchem der erste Faden weiterhin einen zweiten Abschnitt definiert, der dafür eingerichtet ist, an einer anderen zweiten Prothese (11a, 11b) anzugreifen.

6. System nach Anspruch 3, das weiterhin einen zweiten Faden umfasst, der einen zweiten Abschnitt definiert.

7. System nach Anspruch 6, bei dem der zweite Abschnitt dazu eingerichtet ist, an einer zweiten Prothese (11a, 11b) anzugreifen.

8. System nach Anspruch 3, das weiterhin einen dritten Faden umfasst, der einen dritten Abschnitt definiert.

9. System nach Anspruch 8, bei welchem der dritte Abschnitt dafür eingerichtet ist, an einer dritten Prothese anzugreifen.

10. System nach Anspruch 3, das weiterhin einen vierten Faden umfasst, der einen vierten Abschnitt definiert.

11. System nach Anspruch 10, bei welchem der vierte Abschnitt dazu eingerichtet ist, an einer vierten Prothese anzugreifen.

12. System nach Anspruch 3, bei welchem der erste Faden die zweite Prothese (11a, 11b) lösbar angreift.

13. System nach Anspruch 12, bei welchem die erste Prothese (10) zwischen einem nicht ausgedehnten Zustand und einem ausgedehnten Zustand beweglich ist und der Faden an der zweiten Prothese angreift, wenn sich die erste Prothese in einem nicht ausgedehnten Zustand befindet.

14. System nach Anspruch 3, bei welchem der Faden Streben umfasst, die aus der Umfangsebene des Stents deformiert sind.

15. System zum Überbrücken eines Aneurysma, das eine erste Prothese gemäß Anspruch 2 und eine zweite Prothese umfasst, wobei die erste Prothese dafür eingerichtet ist, an einem Teil einer Arterie oberhalb des Aneurysma anzugreifen, wobei die zweite Prothese dafür eingerichtet ist, einen Fluidströmungsweg bereitzustellen, der das Aneurysma überbrückt, und wobei ein proximales Ende der zweiten Prothese dafür eingerichtet ist, lösbar an dem Faden anzugreifen.

16. System zum Überbrücken eines Aneurysma, das eine erste Prothese gemäß Anspruch 2 und eine zweite Prothese umfasst, wobei die erste Prothese eine Matrix von untereinander verbundenen Streben und ein Dichtungsmaterial umfasst, das wenigstens einen Teil der Matrix abdeckt, und wobei die zweite Prothese eine Matrix von untereinander verbundenen Streben und ein Dichtmaterial umfasst, das wenigstens einen Teil der Matrix abdeckt, wobei die erste Prothese dafür eingerichtet ist, an einem Teil einer Arterie oberhalb des Aneurysma anzugreifen, wobei die zweite Prothese dafür eingerichtet ist, einen Fluidströmungsweg zur Verfügung zu stellen, der das Aneurysma überbrückt, und wobei ein proximales Ende der zweiten Prothese dafür eingerichtet ist, lösbar an dem Faden anzugreifen.

17. System nach Anspruch 15, wobei die zweite Prothese weiterhin ein distales Ende umfasst, das dafür eingerichtet ist, an einem Teil der Arterie unterhalb des Aneurysma anzugreifen.

## Revendications

1. Prothèse destinée à étanchéifier un ensemble pour remédier à un anévrisme, comprenant une endoprothèse et un matériau d'étanchéité étant en prise avec au moins une partie de ladite endoprothèse, une partie dudit matériau d'étanchéité étant positionnée au niveau de l'extrémité proximale et à travers le diamètre de la section transversale de ladite prothèse, **caractérisée en ce que** ladite partie dudit le matériau d'étanchéité comprend une cloison (71) et un filet (73a, 73b, 73c, 73d, 73e, 73f, 73g, 73d, 73i) définissant une section (72) comportant moins de matériau que la cloison (71).

2. Prothèse selon la revendication 1 dans laquelle ledit filet est configuré à travers ladite extrémité proximale pour être en prise avec une extrémité proximale d'une seconde prothèse.

3. Système destiné à remédier à un anévrisme comprenant :
■ une première prothèse (10) selon la revendication 2 ; et
■ au moins une seconde prothèse (11a, 11b).

4. Système selon la revendication 3 dans lequel ledit premier filet positionne ladite seconde prothèse (11a, 11b) dans ladite première prothèse (10).

5. Système selon la revendication 3 dans lequel ledit premier filet définit en outre une seconde section configurée pour être en prise avec une autre seconde prothèse (11a, 11b).

6. Système selon la revendication 3 comprenant en outre un second filet définissant une seconde section.

7. Système selon la revendication 6 dans lequel ladite seconde section est configurée pour être en prise avec une seconde prothèse (11a, 11b).

8. Système selon la revendication 3 comprenant en outre un troisième filet définissant une troisième section.

9. Système selon la revendication 8 dans lequel ladite troisième section est configurée pour être en prise avec une troisième prothèse.

10. Système selon la revendication 3 comprenant en outre un quatrième filet définissant une quatrième section.

11. Système selon la revendication 10 dans lequel ladite quatrième section est configurée pour être en prise avec une quatrième prothèse.

12. Système selon la revendication 3 dans lequel ledit premier filet est en prise de manière libérable avec ladite seconde prothèse (11a, 11b).

13. Système selon la revendication 12 dans lequel la première prothèse (10) passe d'un état non dilaté à un état dilaté, et ledit filet est en prise avec ladite seconde prothèse quand la première prothèse est à l'état non dilaté.

14. Système selon la revendication 3, dans lequel le filet comprend des branches déformées hors du plan circonférentiel de l'endoprothèse.

15. Système destiné à ponter un anévrisme comprenant une première prothèse selon la revendication 2 et une seconde prothèse, ladite première prothèse étant adaptée pour être en prise avec une partie d'une artère en amont dudit anévrisme ; ladite seconde prothèse étant configurée pour former un trajet d'écoulement de liquide qui ponte ledit anévrisme et une extrémité proximale de ladite seconde prothèse étant adaptée pour être en prise de manière libérable avec ledit filet.

16. Système destiné à ponter un anévrisme comprenant une première prothèse selon la revendication 2 et une seconde prothèse, ladite première prothèse comprenant une matrice de branches interconnectées et ledit matériau d'étanchéité couvrant au moins une partie de ladite matrice ; et ladite seconde prothèse comprenant une matrice de branches interconnectées et un matériau de greffon couvrant au moins une partie de ladite matrice ; ladite première prothèse étant adaptée pour être en prise avec une partie d'une artère en amont dudit anévrisme, ladite seconde prothèse étant configurée pour former un trajet d'écoulement de liquide qui ponte ledit anévrisme et une extrémité proximale de ladite seconde prothèse étant adaptée pour être en prise de manière libérable avec ledit filet.

17. Système selon la revendication 15 dans lequel ladite seconde prothèse comprend en outre une extrémité distale configurée pour être en prise avec une partie d'une artère en aval dudit anévrisme.
